# EUROPÄISCHE PATENTSCHRIFT

(11) **EP 0 307 491 B1**
(45) Veröffentlichungstag und Bekanntmachung des Hinweises auf die Patenterteilung: **16.06.1993**
(21) Anmeldenummer: 87113496.1
(22) Anmeldetag: 15.09.1987
(51) Int. Cl.: A61B 17/56

(54) **Chirurgisches Gerät zum Einsetzen von Stahlstiften in Knochen**
Surgical instrument for inserting steel rods into bones
Instrument chirurgical pour insérer des tiges en acier dans l'os

(43) Veröffentlichungstag der Anmeldung: 22.03.1989
(73) Patentinhaber: Gebrüder Martin GmbH & Co. KG, D-78532 Tuttlingen (DE)
(72) Erfinder: Vickers, David W., Dr., Brisbane, Q'Ld. 4000 (AU)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- DE-U- 8 400 640
- GB-A- 2 168 920
- US-A- 4 004 338
- US-A- 4 579 023

## Beschreibung

Die Erfindung betrifft eine Zange nach dem Obergriff des Anspruches 1.

Zangen sind in vielfachen Formen und für die verschiedensten Zwecke bekannt. Beispielsweise ist aus der GB-A-2 168 920 eine Zange der eingangs genannten Art mit zwei parallel zueinander angeordneten Schenkeln die an ihren hinteren Enden miteinander verbunden sind und an ihren vorderen Enden mittels eines im vorderen Bereich der Schenkel angelenkten Schwenkhebels einhändig aufeinander zu preßbar sind für die Herstellung von Schmucksteine aufnehmenden Löchern in Fingernägeln bekannt. Im Vergleich hierzu befaßt sich die vorliegende Erfindung mit einer chirurgischen Zange zum Einsetzen von Stahlstiften in Knochen. Zu diesem Zweck ist die aus der GB-A-2 168 920 bekannte Zange ungeeignet, da sie keine Klemmführung für die Stahlstifte aufweist.

Bekannte chirurgische Geräte zum Einsetzen von Stahlstiften in Knochen, die von Hand betätigt werden, besitzen zum Einklemmen der Stahlstifte ein Spannfutter, das mit einer Hand zum Einklemmen und Lösen der Stahlstifte betätigt werden muß, während das chirurgische Gerät mit der anderen Hand gehalten wird. Somit benötigt ein Operateur beide Hände, wenn er beim Einsetzen eines Stahlstiftes in einen Knochen den Stahlstift an einer anderen Stelle einspannen muß.

Derartige chirurgische Geräte sind somit verhältnismäßig umständlich zu handhaben.

Der Erfindung liegt demgegenüber die Aufgabe zugrunde, eine chirurgische Zange für das Einsetzen von Stahlstiften in Knochen zu schaffen, die einfach handzuhaben ist.

Diese Aufgabe wird erfindungsgemäß bei einer Zange der eingangs genannten Art, d.h. nach dem Oberbegriff von Anspruch 1 dadurch gelöst, daß zur Bildung einer chirurgischen Zange zum Einsetzen von Stahlstiften in Knochen, die vorderen Enden der Schenkel als ein eine in Längsrichtung der Schenkel genutete längliche, sich parallel zu den Schenkeln erstreckende Stahlstifthalterung aufweisendes Klemmbackenpaar ausgebildet ist.

Mit der erfindungsgemäßen Zange ist es dem Operateur möglich, sowohl das Festklemmen als auch das Loslassen von Stahlstiften mit einer Hand zu bewirken. Aufgrund der Einhandbedienung der erfindungsgemäßen chirurgischen Zange hat der Operateur somit die andere Hand frei, um den Knochen, in den ein Stahlstift eingesetzt werden soll, festzuhalten.

Um die Handhabung der chirurgischen Zange und insbesondere das Loslassen des Stahlstiftes weiter zu vereinfachen, ist bei einem bevorzugten Ausführungsbeispiel der Erfindung vorgesehen, daß die Schenkel an ihren hinteren Enden mittels eines Schwenkbolzens miteinander verbunden sind und daß eine die Schenkel auseinanderdrückende Feder zwischen ihnen angeordnet ist.

Der Betätigungs- bzw. Schwenkhebel weist vorzugsweise in seinem Anlenkbereich einen mit dem ersten Schenkel in Eingriff stehenden Nocken auf, der beim Verschwenken des Betätigungshebels in Richtung auf die Schenkel die erste Backe auf die zweite Backe zu bewegt.

Weiterhin Kann der Betätigungs- bzw. Schwenkhebel an einer von einer die Klemmbacken umgreifenden Klammer befestigten Anlenkachse angelenktsein.

Ein bevorzugtes Ausführungsbeispiel der Erfindung zeichnet sich dadurch aus, daß die Schenkel einstückig miteinander aus Federstahl hergestellt sind und an ihren hinteren Enden ineinander übergehen, so daß sie ein langgestrecktes U bilden, wobei jeder Schenkel benachbart zum hinteren Übergangsbereich eine zum jeweils anderen Schenkel hin offene Ausnehmung aufweist. Hierdurch wird zum einen die Herstellung des chirurgischen Geräts vereinfacht, ohne daß dadurch die Handhabbarkeit beeinträchtigt wird.

Die die Schenkel auseinanderdrückende Federkraft wird dabei von den im Übergangsbereich miteinander verbundenen Schenkeln selbst erzeugt.

Zum anderen wird durch die vorgesehenen Ausnehmungen bewirkt, daß die Schwenkachse der Schenkel möglichst weit von den Klemmbacken entfernt angeordnet ist, so daß die Klemmflächen der Klemmbacken sowohl beim Festklemmen als auch beim Loslassen eines Stahlstiftes praktisch parallel zueinander verlaufen. Dies ermöglicht ein besonders sicheres und gleichmäßiges Einklemmen eines Stahlstiftes über die gesamte Länge der Klemmflächen.

Um es dem Operateur auf besonders einfache Weise zu ermöglichen, die bereits in den Knochen eingesetzte Länge des Stahlstiftes zu überwachen, ist es bei einem weiteren Ausführungsbeispiel der Erfindung vorgesehen, daß an zumindest einem der Schenkel eine Längenskala angeordnet ist, die sich in Längsrichtung der Schenkel erstreckt.

Bei einer Weiterbildung der Erfindung ist vorgesehen, daß die Klemmbacken in ihren einander gegenüberliegenden Klemmflächen jeweils eine Längsnut aufweisen. Hierdurch wird ein besonders inniger Kontakt zwischen den Klemmflächen der Klemmbacken und dem dazwischen befindlichen Stahlstift über eine beträchtliche Länge der Klemmflächen erreicht, so daß der Stahlstift sicher zwischen den Klemmflächen gehalten ist, ohne daß die Gefahr der Beschädigung seiner Oberfläche besteht.

Bei einer anderen besonders bevorzugten Ausbildung der Erfindung ist vorgesehen, daß benachbart zum hinteren Ende der Klemmbacken einander gegenüberliegende und miteinander in Eingriff bringbare Schneiden an den Schenkeln vorgesehen sind, wobei an den Klemmbacken jeweils ein Biegeelement angeordnet ist, wobei die Biegeelemente einander gegenüberliegen. Hierdurch wird ein chirurgisches Gerät geschaffen, mit dem der Operateur nicht nur Stahlstifte in Knochen einsetzen kann, sondern auch überflüssige Abschnitte des Stahlstiftes abtrennen sowie erforderlichenfalls zurechtbiegen kann. Es wird also ein chirurgisches Gerät geschaffen, das mehrere Funktionen gleichzeitig erfüllen kann, so daß ein umständliches Wechseln der Geräte während einer Operation entfällt.

Bei einem weiteren Ausführungsbeispiel der Erfindung ist vorgesehen, daß der Betätigungshebel einen ersten sich im wesentlichen parallel zu den Schenkeln erstreckenden Abschnitt aufweist, der am hinteren Ende in einen Führungsabschnitt übergeht, der schräg zur Längsrichtung der Schenkel verläuft und der eine Führungsöffnung aufweist, durch die sich die Schenkel hindurcherstrecken, und daß sich an den Führungsabschnitt ein Griffteil des Betätigungshebels anschließt, das auf der anderen Seite einer von einer zwischen den Schenkeln verlaufenden Längsachse und der Schwenkachse der Schenkel gegebenen Ebene liegt wie der den Nocken tragende Abschnitt des Betätigungshebels und das mit den Schenkeln einen spitzen Winkel einschließt.

Hierdurch wird nicht nur die Führung des Betätigungshebels an den Schenkeln auf besonders einfache Weise sichergestellt, sondern es werden gleichzeitig auch die Schenkel in einer Ebene gehalten, so daß der Übergangsbereich der Schenkel ineinander bzw. der die Schenkel verbindende Schwenkbolzen entlastet wird.
Um eine ergonomisch besonders günstige Handhabung des chirurgischen Gerätes sicherzustellen, ist bei einem weiteren Ausführungsbeispiel der Erfindung vorgesehen, daß der Winkel zwischen dem Griffteil des Betätigungshebels und den Schenkeln etwa 10 ° bis 30 ° beträgt.

Ein weiteres Ausführungsbeispiel der Erfindung zeichnet sich dadurch aus, daß die im wesentlichen U-förmige Klammer mit einem Bodensteg an der zweiten Klemmbacke anliegt und daß zwischen den vom Bodensteg abgewandten Enden der Klammerschenkel ein Lagerstift für den Betätigungshebel befestigt ist. Damit wird eine besonders einfache und stabile Anbringung des Betätigungshebels an den Schenkeln des chirurgischen Geräts ermöglicht, die auch ein einfaches Auseinandernehmen des Geräts für Reinigungs- und Sterilisationszwecke zuläßt.

Bei einem weiteren bevorzugten Ausführungsbeispiel der Erfindung ist vorgesehen, daß die Klammer verschiebbar auf die Klemmbacken aufgesteckt ist, wobei die den Klemmflächen gegenüberliegenden Außenflächen der Klemmbacken so gegen die Klemmflächen geneigt sind, daß sich die Klemmbacken nach vorn verjüngen. Hierdurch läßt sich der freie Abstand zwischen den Klemmflächen der Klemmbacken bei entlastetem Betätigungshebel leicht verändern, so daß das chirurgische Gerät an unterschiedliche Dicken von in Knochen einzusetzenden Stahlstiften angepaßt werden kann. Auf diese Weise wird insbesondere auch gewährleistet, daß der Winkei zwischen dem Betätigungshebel und den Schenkeln des chirurgischen Geräts beim Festklemmen verschieden dicker Stahlstifte stets im gleichen Bereich gehalten wird, so daß das Gerät immer auf dieselbe Weise gehandhabt werden kann.

Bei einem anderen Ausführungsbeispiel der Erfindung wird die Anpassung des freien Abstandes der Klemmflächen an unterschiedliche Stahlstiftdicken dadurch erreicht, daß der Lagerstift einstellbar an der Klammer befestigt ist.

Die Erfindung wird im folgenden beispielsweise anhand der Zeichnung näher erläutert; in dieser zeigt:
- Fig. 1: eine schematische Darstellung eines chirurgischen Geräts zum Einsetzen von Stahlstiften in Knochen und
- Fig. 2: eine Draufsicht auf den Klemmbackenbereich eines weiteren Ausführungsbeispiels eines chirurgischen Geräts zum Einsetzen von Stahlstiften in Knochen.

In den beiden Figuren der Zeichnung sind einander entsprechende Teile mit gleichen Bezugszeichen bezeichnet.

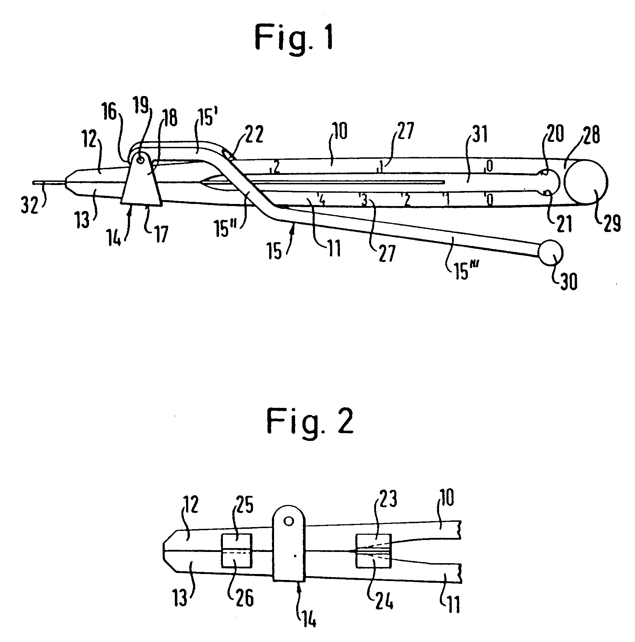

Fig. 1 zeigt ein chirurgisches Gerät zum Einsetzen von Stahlstiften in Knochen mit zwei im wesentlichen parallel zueinander verlaufenden länglichen Schenkeln 10, 11, die an ihrem hinteren, in Fig. 1 rechten Ende einstückig ineinander übergehen und dabei einen Übergangsbereich 28 bilden. Die Schenkel 10, 11 sind dabei aus Federstahl hergestellt, so daß sie aufgrund ihrer Eigenelastizität gegeneinandergedrückt werden können und nach Entlastung in ihre Ausgangsstellung zurückkehren.

Am Übergangsbereich 28 ist ein sich senkrecht zu den Schenkeln 10, 11 erstreckendes Griffstück 29 angeordnet. Benachbart zum Übergangsbereich 28 sind in den Schenkeln 10, 11 Ausnehmungen 20, 21 vorgesehen, die jeweils zum anderen Schenkel 11, 10 hin offen sind und eine nachgebende Stelle der Schenkel 10, 11 bewirken, so daß eine Schwenkachse für die Schenkel 10, 11 benachbart zum Übergangsbereich 28 festgelegt wird.

An ihren vorderen, in Fig. 1 linken Enden weisen die ein im wesentlichen langgestrecktes U bildenden Schenkel 10, 11 Klemmbacken 12, 13 auf, die einander gegenüberliegende Klemmflächen besitzen, in denen sich parallel zur Längsrichtung der Schenkel 10, 11 verlaufende Längsnuten befinden. Die Klemmbacken 12, 13 sind von einer im wesentlichen U-förmigen Klammer 14 umgriffen, die mit ihrem Bodensteg 17 an der unteren Klemmbacke 13 anliegt und zwischen deren vom Bodensteg 17 abgewandten Ende der Klammerschenkel 18 ein Lagerstift 19 angeordnet ist, an dem ein Betätigungshebel 15 angelenkt ist.

Der Betätigungshebel 15 weist einen sich im wesentlichen parallel zu den Schenkeln 10, 11, von Anlenkbereich nach hinten erstreckenden Abschnitt 15' auf, an den sich ein Führungsabschnitt 15'' anschließt, der schräg zu den Schenkeln 10, 11 verläuft und eine Führungsöffnung 22 aufweist, durch die sich die Schenkel 10, 11 hindurchstrecken. An den Führungsabschnitt 15'' schließt sich dann weiter ein Griffteil 15''' an, an dessen hinterem Ende ein senkrecht zum Betätigungshebel 15 angeordnetes Griffstück 30 befestigt ist. Das Griffteil 15''' des Betätigungshebels 15 schließt mit den Schenkeln, 10, 11 einen Winkel von ungefähr 10 ° bis 30 ° ein. Im Anlenkbereich des Betätigungshebels 15 an der Klammer 14 besitzt der Betätigungshebel 15 einen Nocken 16, der mit der oberen Klemmbacke 12 so in Eingriff steht, daß die Klemmbacken 12, 13 zwangsweise gegeneinander gepreßt werden, wenn das Griffteil 15''' des Betätigungshebels 15 auf die Schenkel 10, 11 zu verschwenkt wird.

Zur Übertragung der Klemmkraft vom Betätigungshebel 15 auf die Klemmbacken 12, 13 kann anstelle des Nocken 16 auch eine Hebelanordnung vorgesehen sein.

Zwischen den Schenkeln 10, 11 im Bereich zwischen dem Klemmbacken 12, 13 und den Ausnehmungen 20, 21 ist ein freier Bereich 31 für die Aufnahme des hinteren Endes eines zwischen dem Klemmbacken 12, 13 gehaltenen Stahlstiftes vorgesehen. Benachbart zu den den freien Bereich 31 begrenzenden Innenkanten der Schenkel 10, 11 sind Längenskalen 27 daran vorgesehen, wobei die am oberen oder ersten Schenkel 10 befindliche Längenskala 27 eine Inchteilung aufweist, während die am unteren oder zweiten Schenkel 11 befindliche Längenskala 27 eine Zentimeterteilung besitzt.

Nach Fig. 2 sind benachbart zum hinteren Ende der Klemmbacken 12, 13 an den Schenkeln 10, 11 einander gegenüberliegende und miteinander in Eingriff bringbare Schneiden 23, 24 vorgesehen, so daß das chirurgische Gerät zum Durchtrennen von in Knochen einzusetzenden Stahlstiften verwendbar ist. Zusätzlich sind an den Klemmbacken 12, 13 noch einander zugeordnete Biegeelemente 25, 26 angeordnet, die miteinander eine Biegevorrichtung für Stahlstifte bilden.

Das beschriebene chirurgische Gerät funktioniert wie folgt:
Zunächst wird ein in einen Knochen einzusetzender Stahlstift 32 so zwischen den Klemmbacken 12, 13 angeordnet, daß er parallel zur Längsachse des chirurgischen Geräts innerhalb der Längsnuten zwischen den Klemmflächen der Klemmbacken 12, 13 verläuft und mit einem vorderen Ende zwischen den Klemmbacken 12, 13 hervorsteht. Das hintere Ende des Stahlstiftes 32 ist dabei im freien Bereich 31 zwischen den Schenkeln 10, 11 angeordnet. Nun kann der Operateur das Griffteil 15''' gegen die Schenkel 10, 11 des chirurgischen Geräts drücken, wobei der Nocken 16 die beiden Klemmbacken 12, 13 fest gegeneinanderdrückt, so daß der Stahlstift 32 sicher gehalten ist und sein vorderes Ende in einen Knochen eingesetzt werden kann.

Nachdem das vorne aus dem Klemmbacken 12, 13 hervorstehende Ende des Stahlstiftes 32 in einen Knochen eingesetzt ist, läßt der Operateur den Betätigungshebel 15 los, so daß die Klemmbacken 12, 13 von der auf die Schenkel 10, 11 wirkenden Federkraft auseinandergedrückt werden, wodurch die Klemmung des Stahlstiftes 32 zwischen den Klemmbacken 12, 13 soweit gelockert wird, daß das chirurgische Gerät um einen erforderlichen Betrag längs des Stahlstiftes 32 zurückgezogen werden kann. Nun wird der Betätigungshebel 15 wieder gegen die Schenkel 10, 11 gedrückt, wodurch der Stahlstift 32 an einer anderen Stelle erneut sicher gehalten ist. Der Stahlstift 32 kann nun weiter in den Knochen eingeschoben werden. Dabei ermöglicht es die Längsskala 27 an dem Schenkel 10 oder 11 dem Operateur festzustellen, wieweit das chirurgische Gerät längs des Stahlstiftes 32 zurückgezogen wurde.

Dieser Vorgang wird nun so oft wiederholt, bis eine gewünschte Länge des Stahlstiftes 32 in einen Knochen eingesetzt wurde.

Daraufhin kann der Operateur das chirurgische Gerät vollständig von dem Stahlstift 32 abziehen und das überflüssige, aus dem Knochen hervorstehende Ende des Stahlstiftes mittels der Schneiden 23, 24 abtrennen. Sollte es hierbei erforderlich sein, den Stahlstift 32 umzubiegen, so kann dies ebenfalls mittels der von den Biegeelementen 25, 26 gebildeten Biegevorrichtung durchgeführt werden.

## Patentansprüche

1. Zange mit zwei parallel zueinander angeordneten Schenkeln (10, 11), die an ihren hinteren Enden schwenkbar miteinander verbunden sind und an ihren vorderen Enden (12, 13) mittels eines im vorderen Bereich der Schenkel angelenkten Schwenkhebels (15) einhändig aufeinander zu preßbar sind, dadurch gekennzeichnet, daß zur Bildung einer chirurgischen Zange zum Einsetzen von Stahlstiften (32) in Knochen, die vorderen Enden der Schenkel (10, 11) als ein eine in Längsrichtung der Schenkel genutete, längliche, sich parallel zu den Schenkeln erstreckende Stahlstifthalterung aufweisendes Klemmbackenpaar (12, 13) ausgebildet ist.

2. Chirurgische Zange nach Anspruch 1, dadurch gekennzeichnet, daß der Betätigungs- bzw. Schwenkhebel (15) in seinem Anlenkbereich einen mit dem ersten Schenkel (10) in Eingriff stehenden Nocken (16) aufweist, der beim Verschwenken des Betätigungs- bzw. Schwenkhebels (15) in Richtung auf die Schenkel (10, 11) die erste Backe (12) auf die zweite Backe (13) zu bewegt.

3. Chirurgische Zange nach Anspruch 2, dadurch gekennzeichnet, daß der Betätigungs- bzw. Schwenkhebel (15) an einer von einer die Klemmbacken umgreifenden Klammer (14) befestigten Anlenkachse (19) angelenkt ist.

4. Chirurgische Zange nach einem der vorhergehenden Ansprüche,
dadurch **gekennzeichnet,**
daß die Schenkel (10, 11) an ihren hinteren Enden mittels eines Schwenkbolzens miteinander verbunden sind und daß eine die Schenkel (10, 11) auseinanderdrückende Feder zwischen ihnen angeordnet ist.

5. Chirurgische Zange nach einem der vorhergehenden Ansprüche 1 bis 3,
dadurch **gekennzeichnet,**
daß die Schenkel (10, 11) einstückig miteinander aus Federstahl hergestellt sind und an ihren hinteren Enden ineinander übergehen, so daß sie ein langgestrecktes U bilden, wobei vorzugsweise jeder Schenkel (10, 11) benachbart zum hinteren Übergangsbereich eine zum jeweils anderen Schenkel hin offene Ausnehmung (20, 21) aufweist.

6. Chirurgische Zange nach einem der vorhergehenden Ansprüche,
dadurch **gekennzeichnet,**
daß an zumindest einem der Schenkel (10, 11) eine Längenskala (27) angeordnet ist, die sich in Längsrichtung der Schenkel (10, 11) erstreckt.

7. Chirurgische Zange nach einem der vorhergehenden Ansprüche,
dadurch **gekennzeichnet,**
daß die Klemmbacken (12, 13) in ihren einander gegenüberliegenden Klemmflächen jeweils eine Längsnut aufweisen.

8. Chirurgische Zange nach einem der vorhergehenden Ansprüche,
dadurch **gekennzeichnet,**
daß benachbart zum hinteren Ende der Klemmbacken (12, 13) einander gegenüberliegende und miteinander in Eingriff bringbare Schneiden (23, 24) an den Schenkeln (10, 11) vorgesehen sind und vorzugsweise, daß an den Klemmbacken (12, 13) jeweils ein Biegeelement (25, 26) angeordnet ist, wobei die Biegeelemente (25, 26) einander gegenüberliegen.

9. Chirurgische Zange nach einem der vorhergehenden Ansprüche,
dadurch **gekennzeichnet,**
daß der Betätigungshebel (15) einen ersten sich im wesentlichen parallel zu den Schenkeln (10, 11) erstreckenden Abschnitt (15') aufweist, der am hinteren Ende in einen Führungsabschnitt (15'') übergeht, der schräg zur Längsrichtung der Schenkel (10, 11) verläuft und der eine Führungsöffnung (22) aufweist, durch die sich die Schenkel (10, 11) hindurcherstrecken, daß sich an den Führungsabschnitt (15'') ein Griffteil (15''') des Betätigungshebels (15) anschließt, das auf der anderen Seite einer von einer zwischen den Schenkeln (10, 11) verlaufenden Längsachse und der Schwenkachse der Schenkel (10, 11) gegebenen Ebene liegt wie der den Nocken (16) tragende Abschnitt (15') des Betätigungshebels (15) und das mit den Schenkeln (10, 11) einen spitzen Winkel einschließt, und vorzugsweise, daß der Winkel zwischen dem Griffteil (15''') des Betätigungshebels (15) und den Schenkeln (10, 11) etwa 10° bis 30° beträgt.

10. Chirurgische Zange nach einem der vorhergehenden Ansprüche,
dadurch **gekennzeichnet,**
daß die im wesentlichen U-förmige Klammer (14) mit einem Bodensteg (17) an der zweiten Klemmbacke (13) anliegt und daß zwischen den vom Bodensteg (17) abgewandten Enden der Klammerschenkel (18) ein Lagerstift (19) für den Betätigungshebel (15) befestigt ist und vorzugsweise, daß die Klammer (14) verschiebbar auf die Klemmbacken (12, 13) aufgesteckt ist.

11. Chirurgische Zange nach Anspruch 7,
dadurch **gekennzeichnet,**
daß die den Klemmflächen gegenüberliegenden Außenflächen der Klemmbacken (12, 13) so gegen die Klemmflächen geneigt sind, daß sich die Klemmbacken (12, 13) nach vorn verjüngen.

12. Chirurgische Zange nach einem der vorhergehenden Ansprüche
dadurch **gekennzeichnet,**
daß der die Anlenkachse bildende Lagerstift (19) einstellbar an der Klammer (14) befestigt ist.

## Claims

1. Nipper device having two limbs (10, 11), arranged parallel to one another, which are pivotally connected together at their rear ends and can be pressed together at their front ends (12, 13) in a one-handed operation by means of a pivotal lever (15) pivotally connected in the front region of the limbs, characterised in that, in order to form a surgical nipper for the insertion of steel pins (32) into bones, the front ends of the limbs (10, 11) are formed as a clamping jaw pair (12, 13) having an elongate steel pin holder grooved in the longitudinal direction of the limbs and extending parallel to the limbs.

2. Surgical nipper in accordance with claim 1, characterized in that the actuation or pivotal lever (15) comprises in the region of its pivotal mounting a cam (16) which is engaged with the first limb (10) and displaces the first jaw (12) towards the second jaw (13) on pivoting the actuation or pivotal lever (15) towards the limbs (10, 11).

3. Surgical nipper in accordance with claim, characterized in that the actuation or pivotal lever (15) is pivotally mounted on a pivot axle (19) secured by a bracket (14) which encompasses the clamping jaws.

4. Surgical nipper in accordance with any one of the preceding claims, characterized in that the limbs (10, 11) are mutually connected at their rear ends by means of a pivot bolt; and in that a spring which urges the limbs apart (10, 11) is arranged between them.

5. Surgical nipper in accordance with any one of claims 1 to 3, characterized in that the limbs (10, 11) are realised as a single piece of spring steel and merge into each other at their rear ends, such that they form an elongated U, with each limb (10, 11) being preferably provided with a recess (20, 21) which is located adjacent the rear transition region and is open towards the other limb.

6. Surgical nipper in accordance with any one of the preceding claims, characterized in that a length scale (27) is provided on at least one of the limbs (10, 11), and extends in the longitudinal direction of the limbs (10, 11).

7. Surgical nipper in accordance with any one of the preceding claims, characterized in that the clamping jaws (12, 13) are each provided with an elongate groove in their confronting clamping surfaces.

8. Surgical nipper in accordance with any one of the preceding claims, characterized in that confronting cutters (23, 24) are provided on the limbs (10, 11) in the vicinity of the rear ends of the clamping jaws (12, 13), and can be brought into mutual engagement; and in that a bending element (25, 26) is mounted on each clamping jaw (12, 13), said bending elements (25, 26) facing each other.

9. Surgical nipper in accordance with any one of the preceding claims, characterized in that the actuation lever (15) comprises a first portion (15') which extends substantially parallel to the limbs (10, 11) and merges at the rear end into a guide portion (15'') which extends obliquely to the longitudinal direction of the limbs (10, 11) and is provided with a guide opening (22) through which the limbs (10, 11) extend; in that a grip portion (15''') of the actuation lever adjoins the guide portion (15''), the grip portion being located on the other side of a plane defined by a longitudinal axis extending between the limbs (10, 11) and by the pivot axis of the limbs (10, 11) from the portion (15') of the actuation lever (15) which carries the cam (16), and including with the limbs (10, 11) an acute angle; and preferably in that the angle between the grip portion (15''') of the actuation lever (15) and the limbs (10, 11) amounts to about 10° to 30°.

10. Surgical nipper in accordance with any one of the preceding claims, characterized in that the substantially U-shaped bracket (14) contacts the second clamping jaw (13) by means of a bottom web (17); and in that a bearing pin (19) for the actuation lever (15) is fixed between the ends of the bracket arms (18) remote from the bottom web (17); and preferably in that the bracket (14) is displaceably slipped onto the clamping jaws (12, 13).

11. Surgical nipper in accordance with claim 7, characterized in that the outer surfaces of the clamping jaws (12, 13) opposite from the clamping surfaces are inclined with respect to the clamping surfaces in such a manner that the clamping jaws (12, 13) taper towards the front.

12. Surgical nipper in accordance with any one of the preceding claims, characterized in that the bearing pin (19) which forms the pivot axle is adjustably fixed onto the bracket (14).

## Revendications

1. Pince comprenant deux bras (10, 11) disposés parallèlement l'un à l'autre, qui sont mutuellement reliés en pivotement à leurs extrémités arrières et peuvent être poussés l'un contre l'autre d'une seule main à leurs extrémités avant (12, 13) au moyen d'un levier de basculement (15) articulé dans la région avant des bras, caractérisée en ce que, afin de constituer une pince chirurgicale pour insérer des tiges en acier (32) dans des os, les extrémités avant des bras (10, 11) sont réalisées sous la forme d'une paire de mâchoires de serrage (12, 13) dotées d'une fixation pour tige métallique, allongée et s'étendant parallèlement aux bras, ladite paire présentant une gorge dans la direction longitudinale des bras.

2. Pince chirurgicale selon la revendication 1, caractérisée en ce que le levier d'actionnement ou de basculement (15) comporte dans sa zone d'articulation une came (16) en engagement avec le premier bras (10), ladite came déplaçant la première mâchoire (12) vers la seconde mâchoire (13) lors du basculement du levier d'actionnement ou de basculement (15) en direction des bras (10, 11).

3. Pince chirurgicale selon la revendication 2, caractérisée en ce que le levier d'actionnement ou de basculement (15) est articulé sur un axe d'articulation (19) fixé par un étrier (14) qui chevauche les mâchoires de serrage.

4. Pince chirurgicale selon l'une quelconque des revendications précédentes, caractérisée en ce que les bras (10, 11) sont mutuellement reliés à leurs extrémités arrières au moyen d'un axe de pivotement, et en ce qu'il est disposé entre les bras (10, 11) un ressort qui repousse ceux-ci en écartement.

5. Pince chirurgicale selon l'une quelconque des revendications 1 à 3, caractérisée en ce que les bras (10, 11) sont réalisés ensemble d'une seule pièce en acier ressort et passent continument l'un dans l'autre à leurs extrémités arrières de telle manière qu'ils forment un U allongé, chaque bras (10, 11) présentant de préférence un évidement (20, 21) situé au voisinage de la région de transition arrière et ouvert en direction de l'autre bras.

6. Pince chirurgicale selon l'une quelconque des revendications précédentes, caractérisée en ce qu'il est prévu sur l'un au moins des bras (10, 11) une échelle de longueur (27) qui s'étend dans la direction longitudinale des bras (10, 11).

7. Pince chirurgicale selon l'une quelconque des revendications précédentes, caractérisée en ce les mâchoires de serrage (12, 13) comportent chacune une gorge allongée dans leurs surfaces de serrage qui se font mutuellement face.

8. Pince chirurgicale selon l'une quelconque des revendications précédentes, caractérisée en ce qu'il est prévu des lames (23, 24) sur les bras (10, 11) au voisinage des extrémités arrières des mâchoires de serrage (12, 13), ces lames étant situées en vis-à-vis et pouvant être amenées en engagement l'une avec l'autre, et en ce qu'il est de préférence disposé un élément de coudage (25, 26) sur chacune des mâchoires de serrage (12, 13), lesdits éléments de coudage (25, 26) étant situés face à face.

9. Pince chirurgicale selon l'une quelconque des revendications précédentes, caractérisée en ce le levier d'actionnement (15) comporte un premier tronçon (15') qui s'étend sensiblement parallèlement aux bras (10, 11), ledit tronçon se transformant à l'extrémité arrière en un tronçon de guidage (15'') qui s'étend en oblique par rapport à la direction longitudinale des bras (10, 11) et qui présente une ouverture de guidage (22) traversée par les bras (10, 11), en ce que le tronçon de guidage (15'') est poursuivi par une partie formant poignée (15''') pour le levier d'actionnement (15), ladite partie de poignée étant située de l'autre côté d'un plan défini par un axe longitudinal passant entre les bras (10, 11) et par l'axe de pivotement des bras (10, 11) par rapport au tronçon (15') du levier d'actionnement (15) qui porte la came (16), et la partie formant poignée (15'') formant avec les bras (10, 11) un angle aigu, et en ce que de préférence l'angle entre la partie formant poignée (15''') pour le levier d'actionnement (15) et les bras (10, 11) est compris entre environ 10° et 30°.

10. Pince chirurgicale selon l'une quelconque des revendications précédentes, caractérisée en ce l'étrier (14) qui présente sensiblement la forme d'un U est en contact contre la seconde mâchoire de serrage (13) par l'intermédiaire d'un voile de fond (17), et en ce que une tige de montage (19) pour le levier d'actionnement (15) est fixée entre les extrémités des bras (18) de l'étrier éloignées du voile de fond (17), et de préférence en ce que l'étrier (14) est engagé sur les mâchoires de serrage (12, 13) de manière à pouvoir être déplacé.

11. Pince chirurgicale selon la revendication 7, caractérisée en ce que les faces extérieures des mâchoires de serrage (12, 13) qui sont opposées aux surfaces de serrage sont inclinées par rapport aux surfaces de serrage de telle manière que les mâchoires de serrage (12, 13) s'amincissent vers l'avant.

12. Pince chirurgicale selon l'une quelconque des revendications précédentes, caractérisée en ce la tige de montage (19) qui forme l'axe d'articulation est fixée de façon réglable sur l'étrier (14).
